# EUROPEAN PATENT SPECIFICATION

(11) **EP 2 413 893 B1**
(45) Date of publication and mention of the grant of the patent: **10.09.2014**
(21) Application number: 09789566.8
(22) Date of filing: 01.04.2009
(51) Int. Cl.: A61K 8/19, A61Q 11/00, A61K 8/34, A61K 8/46, A61K 8/73

(54) **DESENSITIZING DENTIFRICE**
DESENSIBILISIERENDE ZAHNPUTZMITTEL
DENTIFRICE DÉSENSIBILISANT

(43) Date of publication of application: 08.02.2012
(73) Proprietor: Colgate-Palmolive Company, New York, NY 10022 (US)
(72) Inventor: MIRAJKAR, Yelloji-rao, K., Piscataway NJ 08854 (US); MATTAI, Jairajh, Piscataway NJ 08854 (US); PRENCIPE, Michael, Princeton Junction NJ 08550 (US)
(74) Representative: Jenkins, Peter David
(86) International application number: PCT/US2009/039201
(87) International publication number: WO 2010/114541

(56) References cited:
- EP-A- 0 278 744
- EP-A- 0 803 243
- US-A- 5 188 821
- US-A1- 2004 076 591
- THORSTEINN LOFTSSON ET AL.: JOURNAL OF PHARMACEUTICAL SCIENCES, vol. 88, no. 12, December 1999 (1999-12), pages 1254-1258, XP002560252
- GROVE C ET AL: "IMPROVING THE AQUEOUS SOLUBILITY OF TRICLOSAN BY SOLUBILIZATION, COMPLEXATION, AND IN SITU SALT FORMATION" JOURNAL OF COSMETIC SCIENCE, SOCIETY OF COSMETIC CHEMISTS, NEW YORK, NY, US, vol. 54, no. 6, 1 November 2003 (2003-11-01), pages 537-550, XP008062569 ISSN: 1525-7886

## Description

### BACKGROUND OF THE INVENTION

Dentinal hypersensitivity is defined as acute, localized tooth pain in response to physical stimulation as by thermal (hot or cold), osmotic, tactile, and/or a combination of thermal, osmotic and tactile stimulation of the exposed dentin.

It is known to the art that potassium salts are effective in the treatment of dentinal hypersensitivity. For example, the prior art discloses that toothpastes containing potassium salts, such as potassium nitrate, potassium chloride or potassium phosphates, desensitize the teeth after tooth brushing for several weeks. It is reported that an elevation in the extracellular potassium concentration in the vicinity of pulpal nerves underlying sensitive dentin is responsible for the therapeutic desensitizing effect of topically applied oral products which contain potassium salts. Due to passive diffusion of potassium ion into and out of the open dentine tubules, repeated application of the active ingredient is necessary to build up the necessary concentration in the vicinity of the pulpal nerves.

In addition to treating dental hypersensitivity, it is desirable to provide dentifrice to control dental plaque. Plaque adheres tenaciously at the points of irregularity or discontinuity, e.g., on rough calculus surfaces, at the gum line and the like. Besides being unsightly, plaque is implicated in the occurrence of gingivitis and other forms of periodontal disease.

A wide variety of antibacterial agents have been suggested in the art to retard plaque formation and the oral infections and dental disease associated with plaque formation. For example, halogenated hydroxydiphenyl ether compounds such as triclosan are well known to the art for their antibacterial activity and have been used in oral compositions to counter plaque formation by bacterial accumulation in the oral cavity. The effectiveness of the antibacterial agent is dependent upon its delivery to and uptake by teeth and soft tissue areas of the gums.

In some current commercial dentifrice formulations, triclosan is solubilized in the presence of sodium lauryl sulfate (SLS), which is a surfactant to provide foaming and cleaning benefits, in order for the triclosan to be able effectively to deliver the benefits of antiplaque efficacy and protection against gingivitis.

However, potassium salts, such as potassium chloride or potassium phosphates, are incompatible with sodium lauryl sulfate due to the formation of potassium lauryl sulfate, a molecule which is insoluble in water. In addition, this combination results in a dentifrice which exhibits very little foaming.

Hydroxypropyl-beta-cyclodextrin (HPβCD) is a molecule known for its ability to increase the solubility of hydrophobic ingredients, including triclosan, in water. However, it has been reported that the combined use of two solubilizing agents, a surfactant (such as sodium lauryl sulfate) and cyclodextrin, results in a much lower solubility of hydrophobic ingredients than when either one is used along at the same concentration.

There is therefore a need in the art to provide means whereby the delivery to and uptake by dental tissue of antibacterial compounds contained in oral compositions containing potassium ions to provide therapeutic efficacy of the antibacterial agent with a desensitizing dentifrice.

There is also a need in the art for a dentifrice composition which can provide both therapeutic and desensitizing benefits.

There is furthermore a need in the art for a dentifrice composition which can provide both therapeutic benefits, from triclosan, and desensitizing benefits, from a potassium salt, and yet provide good triclosan solubility and an acceptable level of foaming.

### BRIEF SUMMARY OF THE INVENTION

The present invention relates to a desensitizing dentifrice, and in particular to such a dentifrice additionally having an anti-bacterial efficacy.

In a first aspect, this invention provides an oral composition comprising an an orally acceptable vehicle, an effective therapeutic amount of an antibacterial compound, an effective therapeutic amount of a source of potassium cations, an anionic surfactant and a cyclodextrin, wherein the source of potassium cations comprises a water soluble potassium salt selected from potassium nitrate, potassium citrate, potassium chloride, potassium bicarbonate and potassium oxalate, which potassium salt is present in the composition at a concentration of 3 wt% to 15 wt% based on the weight of the composition, and wherein the anionic surfactant is sodium lauryl sulfate, present in the composition at a concentration of 0.5 wt% to 2 wt% based on the weight of the composition. Also described herein is an oral composition comprising an orally acceptable vehicle for such composition, an effective therapeutic amount of an antibacterial compound, an effective therapeutic amount of a source of potassium cations, an anionic surfactant and a cyclodextrin.

The antibacterial compound may comprise a halogenated diphenyl ether, typically 2,4,4'-trichloro-2'-hydroxy-diphenyl ether. The 2,4,4'-trichloro-2'-hydroxydiphenyl ether may be present in the composition at a concentration of 0.05 wt. % to 2 wt. % based on the weight of the composition.

The source of potassium cations comprises a water soluble potassium salt, such as potassium chloride. The potassium salt is present in the composition at a concentration of 3 wt. % to 15 wt. % based on the weight of the composition.

The anionic surfactant is sodium lauryl sulfate. The sodium lauryl sulfate may be present in the composition at a concentration of 0.5 wt. % to 2 wt. % based on the weight of the composition.

The cyclodextrin is typically hydroxypropyl-beta-cyclodextrin. The cyclodextrin may be present in the composition at a concentration of 1 wt. % to 15 wt. % based on the weight of the composition.

The oral composition is typically a dentifrice.

Also disclosed herein is a dentifrice composition comprising an orally acceptable vehicle for such composition, 0.05 wt. % to 2 wt. % of an antibacterial compound comprising 2,4,4'-trichloro-2'-hydroxy-diphenyl ether, 0.5 wt. % to 20 wt. % qf a source of potassium cations, 0.5 wt. % to 2 wt. % of an anionic surfactant comprising sodium lauryl sulfate and 1 wt. % to 15 wt. % hydroxypropyl-beta-cyclodextrin, all weights being based on the weight of the composition.

In a third aspect, this invention provides a solubilizing agent for 2,4,4'-trichloro-2'-hydroxy-diphenyl ether in an oral care composition, the solubilizing agent comprising, in combination, sodium lauryl sulfate and hydroxypropyl-beta-cyclodextrin.

In a further aspect, this invention provides a method of solubilizing a halogenated diphenyl ether in a dentifrice composition comprising a source of potassium ions, wherein the source of potassium cations comprises a water soluble potassium salt selected from potassium nitrate, potassium citrate, potassium chloride, potassium bicarbonate and potassium oxalate, which potassium salt is present in the composition at a concentration of 3 wt% to 15 wt% based on the weight of the composition, and sodium lauryl sulfate present in the composition at a concentration of 0.5 wt% to 2 wt% based on the weight of the composition, the method comprising adding a cyclodextrin to the composition. Also disclosed herein is method of solubilizing a halogenated diphenyl ether in a dentifrice composition comprising a source of potassium ions and sodium lauryl sulfate, the method comprising adding a cyclodextrin to the composition. The halogenated diphenyl ether may comprise 2,4,4'-trichloro-2'-hydroxy-diphenyl ether. The cyclodextrin may comprise hydroxypropyl-beta-cyclodextrin.

Also described herein is an oral composition comprising an orally acceptable vehicle for such composition, an effective therapeutic amount of an antibacterial compound comprising a halogenated diphenyl ether, an effective therapeutic amount of an anti-hypersensitivity agent comprising a potassium salt, and a solubilizing agent for the antibacterial compound, the solubilizing agent comprising, in combination, sodium lauryl sulfate and a cyclodextrin.

The halogenated diphenyl ether may comprise 2,4,4'-trichloro-2'-hydroxydiphenyl ether. The 2,4,4'-trichloro-2'-hydroxy-diphenyl ether may be present in the composition at a concentration of 0.05 wt. % to 2 wt. % based on the weight of the composition.

The potassium salt may be water soluble. The potassium salt may be at least one of potassium chloride and potassium nitrate.

The solubilizing agent may comprise 0.5 wt. % to 2 wt. % of sodium lauryl sulfate and 1 wt. % to 15 wt. % hydroxypropyl-beta-cyclodextrin, based on the weight of the composition.

The present invention also provides a method for the treatment and prevention of bacterial plaque accumulation with reduced discomfort and pain associated with dentinal hypersensitivity comprising administering to the oral cavity an oral composition according to the present invention.

The present invention is predicated on the finding by the present inventors that the incorporation of a cyclodextrin, in particular a substituted cyclodextrin, more particularly a hydroxyalkyl-beta-cyclodextrin, most particularly hydroxypropyl-beta-cyclodextrin, into aqueous solutions containing an anionic surfactant, in particular sodium lauryl sulfate, a potassium salt and a halogenated diphenyl ether, such as 2,4,4'-trichloro-2'-hydroxy-diphenyl ether ("Triclosan") unexpectedly reduced the formation of precipitates and exhibited increased triclosan solubility and increased overall foaming. These technical effects are also exhibited in a dentifrice composition comprising these components. The increased triclosan solubility can result in a corresponding increase in the efficacy of triclosan in a dentifrice composition.

### DETAILED DESCRIPTION OF THE INVENTION

The present invention provides an oral composition, such as a dentifrice, comprising an orally acceptable vehicle for such composition, an effective therapeutic amount of an antibacterial compound, an effective therapeutic amount of a source of potassium cations, an anionic surfactant and a cyclodextrin. The composition can exhibit increased uptake by dental tissue of antibacterial compounds contained therein and eliminates or substantially reduces the discomfort and pain associated with dentinal hypersensitivity.

The present inventors have found that a combination of an anionic surfactant, which is normally incompatible with potassium salts because a precipitate would tend to form, with a cyclodextrin can act as a solubilizing agent for the anti-bacterial compound even in the presence of the potassium salt. The combination of the anionic surfactant and the cyclodextrin can be employed in dentifrice compositions containing an anti-bacterial compound and, as an anti-hypersensitivity agent, a potassium salt. By retaining the anti-bacterial compound in solution, so that it is not precipitated in the composition, the delivery and uptake of the anti-bacterial compound by teeth has been found by the inventors to be greatly enhanced.

The oral composition contains a mixture of an anionic surfactant and a cyclodextrin. Useful cyclodextrins include alpha-CD, beta-CD, gamma-CD, hydroxypropyl-alpha-cyclodextrin (HP-alpha-CD), HP-beta-CD, HP-gamma-CD and trimethyl-beta-CD (TM-beta-CD), particularly hydroxypropyl-beta-cyclodextrin.

In one embodiment, the composition contains an anionic surfactant having a concentration ranging 0.5 wt. % to 2 wt. % based on the weight of the composition and a cyclodextrin having a concentration ranging 1 wt. % to 15 wt. % based on the weight of the composition. In another embodiment, the composition contains an anionic surfactant having a concentration ranging from 1.25 wt. % to 1.75 wt. % based on the weight of the composition and a cyclodextrin having a concentration ranging 3 wt. % to 10 wt. % based on the weight of the composition. In one embodiment, the anionic surfactant is sodium lauryl sulfate ("SLS") and the cyclodextrin is hydroxypropyl-beta-cyclodextrin.

In the presence of potassium ions, the SLS anionic surfactant/hydroxypropyl-beta-cyclodextrin mixture of one embodiment acts as a solubilizing agent for the antibacterial compound, in particular when the antibacterial compound comprises a halogenated diphenyl ether, such as 2,4,4'-trichloro-2'-hydroxy-diphenyl ether, so that the antibacterial compound remains in solution, which is essential for the effective delivery of the antibacterial compound. This is unlike certain oral compositions containing sodium lauryl sulfate which causes the antibacterial compound to precipitate from solutions containing potassium ions.

Anti-bacterial agents useful in this invention include water insoluble noncationic compounds, particularly halogenated diphenyl ethers, phenol and homologs, mono- and poly-alkyl and aromatic halophenols, bisphonolic compounds, and resorcinol and its derivatives. More particularly, halogenated diphenyl ethers that are useful for the preparation of the oral care compositions of the present invention, based on considerations of antiplaque effectiveness and safety, include 2,4,4'-trichloro-2'-hydroxy-diphenyl ether (triclosan) and 2,2'-dihydroxy-5,5'-dibromo-diphenyl ether. In one embodiment, the antibacterial compound is 2,4,4'-trichloro-2'-hydroxy-diphenyl ether ("Triclosan").

Antibacterial compounds including phenol and its homologs, mono and polyalkyl and aromatic halophenols, resorcinol and its derivatives and bisphenolic compounds are fully disclosed in U.S. Pat. No. 5,368,844, . Phenolic compounds include n-hexyl resorcinol and 2,2'-methylene bis (4-chloro-6-bromophenol).

The halogenated diphenyl ether or phenolic antibacterial compound is present in the oral composition of the present invention in an effective therapeutic amount. In one embodiment, the effective therapeutic amount ranges of 0.05 wt. % to 2 wt. % based on the weight of the composition. In another embodiment, the effective therapeutic amount ranges of 0.1 wt. % to 1% wt. % based on the weight of the oral composition.

The source of desensitizing potassium ion is generally a water soluble potassium salt including potassium nitrate, potassium citrate, potassium chloride, potassium bicarbonate and potassium oxalate. In one embodiment, the water soluble potassium salt is potassium nitrate. In another embodiment, the water soluble potassium salt is potassium chloride. In such embodiment, the potassium salt is generally incorporated in one or more of the dentifrice components at a concentration ranging of 0.5 wt. % to 20 wt. % based on the weight of the composition. In another such embodiment, the potassium salt is generally incorporated in one or more of the dentifrice components at a concentration ranging of 3 wt. % to 15 wt. % based on the weight of the composition.

In the preparation of an oral composition in accordance with the practice of the present invention, an orally acceptable vehicle including a water-phase with humectant is present. The humectant includes one or more of glycerin, sorbitol, propylene glycol and mixtures thereof. In one embodiment, water is present in amount of at least 10 wt. % based on the weight of the composition. In another embodiment, water is present in an amount of at least 30 wt. % to 60 wt. % based on the weight of the composition. In yet another embodiment, the humectant concentration typically totals 40-60 wt. % of the oral composition.

Dentifrice compositions such as toothpastes and gels also typically contain polishing materials. In one embodiment, the polishing material includes crystalline silica, having a particle size of up to 20 microns, such as commercially available Zeodent 115, or Zeodent 165, silica gel or colloidal silica. In another embodiment, the polishing material includes compositions such as complex amorphous alkali metal aluminosilicates, hydrated alumina, sodium metaphosphate, sodium bicarbonate, calcium carbonate, calcium pyrophosphate, dicalcium phosphate and dicalcium phosphate dihydrate. In one embodiment, the polishing material is included in semisolid or pasty dentifrice compositions, of the present invention, in an amount of 15 wt. % to 60 wt. %. In another embodiment, the composition of the present invention includes polishing material having concentrations ranging of 20 wt. % to 55 wt. % based on the weight of the composition.

Dentifrices prepared in accordance with the present invention typically contain a natural or synthetic thickener. Suitable thickeners include Irish moss, i-carrageenan, gum tragacanth, starch, polyvinylpyrrolidone, hydroxyethypropyl cellulose, hydroxybutyl methyl cellulose, hydroxypropyl methylcellulose, hydroxyethyl cellulose, sodium carboxymethyl cellulose (sodium CMC) and colloidal silica. In one embodiment, the thickener concentration ranges of 0.1 wt. % to 5 wt. % based on the weight of the composition. In another embodiment, the thickener concentration ranges of 0.5 wt. % to 2 wt. % based on the weight of the composition.

The oral composition may also contain a source of fluoride ions, or fluoride-providing compound, as an anti-caries agent. In one embodiment, the fluoride ion composition is provided in an amount sufficient to supply fluoride ions ranging from 25 ppm to 5,000 ppm of the oral composition In another embodiment, the fluoride ion composition is provided in an amount sufficient to supply fluoride ions ranging from 500 to 1500 ppm of the oral composition. Representative fluoride ion providing compounds include inorganic fluoride salts, such as soluble alkali metal salts, for example, sodium fluoride, potassium fluoride, sodium fluorosilicate, ammonium fluorosilicate and sodium monofluorophosphate, as well as tin fluorides, such as stannous fluoride and stannous chloride.

An antibacterial enhancing agent may also be included in the oral composition. In one embodiment, the antibacterial enhancing agent is incorporated in the compositions of the present invention in weight amounts ranging of 0.05 wt. % to 3 wt. % based on the weight of the composition. In another embodiment, the antibacterial enhancing agent is incorporated in the compositions of the present invention in weight amounts ranging of 0.1 wt. % to 2 wt. % based on the weight of the composition.

The use of antibacterial enhancing agents in combination with antibacterial agents such as triclosan is known to the art, as for example U.S. Pat. No. 5,188,821 and U.S. Pat. No. 5,192,531. In one embodiment, the antibacterial enhancing agent is an anionic polymeric polycarboxylate having a molecular weight ranging from 1,000 to 5,000,000 g/mole. In another embodiment, the antibacterial enhancing agent is an anionic polymeric polycarboxylate having a molecular weight ranging from 30,000 to 2,500,000 g/mole. In one embodiment, the anionic polymeric polycarboxylates are generally employed in the form of their free acids. In another embodiment, the anionic polymer polycarboxylates are employed in the form of a partially or fully neutralized water soluble alkali metal salt, e.g., sodium, potassium or ammonium salts. In one embodiment, the antibacterial enhancing agents include 1:4 to 4:1 copolymers of maleic anhydride or acid with another polymerizable ethylenically unsaturated monomer. In one such embodiment, the maleic anhydride copolymer includes a methyl vinyl ether/maleic anhydride copolymer having a molecular weight ("M.W.") ranging from 30,000 to abut 2,500,000 g/mole. These copolymers are commercially available, for example, under the trademark Gantrez, including Gantrez® AN 139 (M.W. 1,000,000 g/mole), Gantrez® AN 119 (M.W. 200,000 g/mole), Gantrez® ES-225 (M.W. 100,000 to 150,000 g/mole) and Gantrez® S-97 Pharmaceutical Grade (M.W. 2,000,000 g/mole), of ISP Corporation.

Any suitable flavoring or sweetening material may also be employed in the preparation of the oral compositions of the present invention. Examples of suitable flavoring constituents include flavoring oils, e.g. oil of spearmint, peppermint, wintergreen, clove, sage, eucalyptus, marjoram, cinnamon, lemon, orange, and methyl salicylate. Suitable sweetening agents include sucrose, lactose, maltose, xylitol, sodium cyclamate, aspartyl phenyl alanine methyl ester, saccharine and the like. Suitably, flavor and sweetening agents may each or together constitute 0.1 wt. % to 5 wt. % of the oral composition.

Various other materials may be incorporated in the oral preparations of this invention such as whitening agents, including urea peroxide, calcium peroxide, and hydrogen peroxide, preservatives, vitamins such as vitamin B6, B12, E and K, silicones, chlorophyll compounds and potassium salts for the treatment of dental hypersensitivity such as potassium nitrate and potassium citrate. These agents, when present, are incorporated in the compositions of the present invention in amounts which do not substantially adversely affect the properties and characteristics desired.

The present invention also provides for a method for the treatment and prevention of bacterial plaque accumulation with reduces discomfort and pain associated with dentinal hypersensitivity by administering to the oral cavity the oral composition discussed herein.

The manufacture of the oral composition of the present invention is accomplished by any of the various standard techniques for producing such compositions. To make a dentifrice, a vehicle is prepared containing humectant, for example, one or more of glycerin, glycerol, sorbitol, and propylene glycol, thickener agents and antibacterial agent such as triclosan, and the vehicle and the anionic surfactant, such as SLS, and the cyclodextrin are added, followed by blending in of a polishing agent, as well as fluoride salts, with the pre-mix. Finally, flavoring agent, is admixed and the pH is adjusted to between 6.8 to 7.

The following examples are further illustrative of the present invention, but it is understood that the invention is not limited thereto. All amounts and proportions referred to herein and in the appended claims are by weight, unless otherwise indicated.

### EXPERIMENTAL EXAMPLES

### Example 1

Four aqueous solutions containing sodium lauryl sulfate (SLS), potassium chloride and hydroxypropyl-beta-cyclodextrin, were prepared. Their composition with respect to these ingredients (all amounts are in wt %) together with their appearance and foaming characteristics, are shown in Table 1 below.

**Table 1: Aqueous solutions containing Sodium Lauryl Sulfate, Potassium Chloride and Hydroxypropyl-beta-cyclodextrin and their physical appearance and foaming attributes.**

| Ingredient | *Formula 1* | *Formula 2* | *Formula 3* | *Formula 4* |
|---|---|---|---|---|
| Sodium Lauryl Sulfate | 1.0% | 1.0% | 1.0% | 1.0% |
| Potassium Chloride | 1.0% | 1.0% | 1.25% | 1.5% |
| Hydroxypropyl-beta-cyclodextrin | ---- | 10.0% | 10% | 10% |
| Water | 98% | 88% | 87.75% | 87.5% |
| Appearance | Turbid and precipitate | Clear | Clear | Slightly Turbid |
| Foaming | Very little | Significant amount | Moderate foam | Moderate Foam |

Formula No.1, containing 1% SLS and 1% KCl, showed the formation of a thick precipitate with little foaming. To demonstrate the impact of the cyclodextrin on precipitate formation and foaming, formula nos. 2, 3 and 4 were tested. These formulas contained increasing levels of KCl (from 1% to 1.5% KCl) along with 1% SLS and 10% Hydroxypropyl-beta-cyclodextrin. The solutions remained clear up to a KCl concentration of 1.25%. Beyond that, at 1.5% KCl, the solution became slightly turbid; however, the turbidity was significantly reduced compared to Formula no.1 without any cyclodextrin. Additionally, the solutions containing cyclodextrin exhibited higher amounts of foam compared to Formula no.1 without any cyclodextrin.

A further aqueous solution, Formula no. 5, containing 1.5% SLS and 3.75% KCl (representing typical concentrations of these components in dentifrices), is shown in Table 2. When these agents are present in water, the solution appeared turbid with precipitate formation, and exhibited no foaming. A similar solution with the addition of 10% Hydroxypropyl-beta-cyclodextrin (formula no. 6, Table 2), however, appeared less turbid, with a lower amount of precipitate, and exhibited increased foaming.

**Table 2: Aqueous solutions containing Sodium Lauryl Sulfate, Potassium Chloride and Hydroxypropyl-beta-cyclodextrin and their physical appearance and foaming attributes.**

| Ingredient | *Formula 5* | *Formula 6* |
|---|---|---|
| Sodium Lauryl Sulfate Powder | 1.50% | 1.50% |
| Potassium Chloride | 3.75% | 3.75% |
| Hydroxypropyl-beta-cyclodextrin | ---- | 10% |
| Water | 94.75% | 84.75% |
| Appearance | Turbid and precipitate | Turbid and less precipitate |
| Foaming | No foaming | Some foaming |

The reduced formation of precipitate and increased foaming with hydroxypropyl-beta-cyclodextrin was attributed by the present inventors to the ability of hydroxypropyl-beta-cyclodextrin to interfere with precipitation, leading to more free SLS. If this attribution was true, since the solubility of triclosan is dependent on the availability of SLS in solution, the addition of hydroxypropyl-beta-cyclodextrin was postulated by the present inventors to be likely to increase the solubility of triclosan.

### Example 2

Results from the following experiments confirmed the increased solubility of triclosan in the presence and absence of KCl due to the addition of hydroxypropyl-beta-cyclodextrin.

In the first set of experiments, the solubility of triclosan in the presence/absence of KCl was measured using a filtration methodology in which the filtrate was analyzed for triclosan. Solubility results, shown in Table 3, suggest that addition of SLS increased the solubility of triclosan (compared to the control with no SLS). Addition of KCl showed precipitation and the analysis of the filtrate showed decreased solubility of triclosan (compared with 1.5% SLS alone). These results were as predicted based on the prior results of Experiment 1.

**Table 3: Solubility of triclosan in aqueous solution containing either sodium lauryl sulfate alone or in combination with potassium chloride.**

| Description | Appearance | Triclosan solubility, % |
|---|---|---|
| 0% SLS - 0% | Suspension and no precipitate | 0.012 |
| 1.5% SLS - 0% KCl | Suspension and no precipitate | 0.330% |
| 1.5% SLS - 3.75% KCl | Turbid and thick precipitate | 0.110 |

In the following experiments, triclosan solubility was measured with increasing concentrations of hydroxypropyl-beta-cyclodextrin (no SLS, no KCl). The results are shown in Table 4, and clearly showed that triclosan solubility increased in a dose-dependent manner.

**Table 4: Solubility of triclosan in aqueous solutions containing only Hydroxypropyl-beta-cyclodextrin**

| Description | Triclosan solubility, % |
|---|---|
| 0% HPβCD in water | 0.012 |
| 10% HPβCD in water | 0.510 |
| 15% HPβCD in water | 0.680 |
| 20% HPβCD in water | 0.710 |

In the next experiments, triclosan solubility was measured in the presence of hydroxypropyl-beta-cyclodextrin along with SLS and KCl. The results shown in Table 5 clearly showed increased solubility of triclosan in the presence of cyclodextrin; the dissolved amount also increased in the dose-dependent manner.

**Table 5: Solubility of triclosan in aqueous solutions containing SLS/KCl and Hydroxypropyl-beta-cyclodextrin.**

| Description | Triclosan solubility, % |
|---|---|
| 0% SLS | 0.012 |
| 1.5% SLS - 3.75% KCl | 0.110 |
| 1.5% SLS - 3.75 KCl - 10% HPbCD | 0.190 |
| 1.5% SLS - 3.75 KCl - 20% HPbCD | 0.380 |

These results of these experiments suggest that by incorporating hydroxypropyl-beta-cyclodextrin, the solubility of triclosan in formulations can be improved.

### Example 3: Comparative Example 1

Dentifrice formulations were prepared containing typical active constituents and orally acceptable vehicles.

In accordance with Example 1, a dentifrice composition had the formulation shown in Table 6, and included, inter alia, triclosan, SLS, KCl, and hydroxypropyl-beta-cyclodextrin in the amounts shown. A comparative dentifrice composition had the formulation shown in Table 6 for Comparative Example 1, and included, inter alia, triclosan, SLS, and KCl in the amounts shown, but did not include hydroxypropyl-beta-cyclodextrin.

The compositions were tested to determine the solubility of triclosan in the respective compositions and the results are shown in Table 7.

From Table 7 it may be clearly seen that the solubility of triclosan increased with the addition of the hydroxypropyl-beta-cyclodextrin to the dentifrice composition. The solubilized amount of triclosan from the dentifrice composition of Example 1 was measurable even after three-fold dilution with water, as shown in Table 7.

**Table 6: Formulations of prototype dentifrices containing SLS and KCl, with and without Hydroxypropyl-beta-cyclodextrin.**

| Ingredients (wt%) | Comparative Example 1 (without HPβCD) | Example 1 (with 5% HPβCD) |
|---|---|---|
| Glycerin | 29.52 | 29.52 |
| Water | 24.36 | 19.36 |
| Potassium Chloride | 3.75 | 3.75 |
| Sodium Fluoride | 0.32 | 0.32 |
| Sodium Saccharin | 0.40 | 0.40 |
| SLS | 1.50 | 1.50 |
| PVM/MA polymer (13% Solution) | 15.0 | 15.0 |
| Sodium Hydroxide - 50% Solution | 1.20 | 1.20 |
| Dental Cream Flavor | 1.15 | 1.15 |
| KCl | 3.75 | 3.75 |
| Triclosan | 0.30 | 0.30 |
| Silicas | 18.75 | 18.75 |
| Hydroxypropyl-β-cyclodextrin | Nil | 5 |
| Total | 100 | 100 |

**Table 7: Solubility of triclosan in dilute solutions of formulations of prototype dentifrices containing SLS and KCl, with and without Hydroxypropyl-beta-cyclodextrin.**

| | Solubility of Triclosan, ppm | |
|---|---|---|
| Dilution | Comparative Example 1 (without HPβCD) | Example 1 (with 5% HPβCD) |
| 1-1 | 120 | 560 |
| 1-2 | 0 | 470 |
| 1-3 | 0 | 160 |

Taken together, these results clearly demonstrate the unexpected benefit of hydroxypropyl-beta-cyclodextrin towards increasing the solubility of triclosan in the presence of KCl and SLS. While the potassium salt could deliver the desensitizing benefit, triclosan, now readily available in dissolved form, could potentially provide the other therapeutic benefits.

The invention is not to be limited in scope by the specific embodiments disclosed in the examples, which are intended as illustrations of a few aspects of the invention, and any embodiments, which are functionally equivalent, are within the scope of this invention. Indeed, various modifications of the invention in addition to those shown and described herein will become apparent to those skilled in the art and are intended to fall within the appended claims.

## Claims

1. An oral composition comprising an orally acceptable vehicle, an effective therapeutic amount of an antibacterial compound, an effective therapeutic amount of a source of potassium cations, an anionic surfactant and a cyclodextrin, wherein the source of potassium cations comprises a water soluble potassium salt selected from potassium nitrate, potassium citrate, potassium chloride, potassium bicarbonate and potassium oxalate, which potassium salt is present in the composition at a concentration of 3 wt% to 15 wt% based on the weight of the composition, and wherein the anionic surfactant is sodium lauryl sulfate, present in the composition at a concentration of 0.5 wt% to 2 wt% based on the weight of the composition.

2. The oral composition of claim 1, wherein said antibacterial compound comprises a halogenated diphenyl ether, optionally wherein the halogenated diphenyl ether comprises 2,4,4'-trichloro-2'-hydroxy-diphenyl ether, further optionally wherein the 2,4,4'-trichloro-2'-hydroxydiphenyl ether is present in the composition at a concentration of 0.05 wt. % to 2 wt% based on the weight of the composition.

3. The oral composition of claim 1, wherein the source of potassium ions is potassium chloride.

4. The oral composition of claim 1, wherein the cyclodextrin is hydroxypropyl-beta-cyclodextrin.

5. The oral composition of claim 1, wherein the cyclodextrin is present in the composition at a concentration of 1 wt. % to 15 wt. % based on the weight of the composition.

6. The oral composition of claim 1, wherein said composition is a dentifrice.

7. The dentifrice composition of claim 6 wherein said antibacterial compound comprises 2,4,4'-trichloro-2'-hydroxy-diphenyl ether, optionally present in the composition at a concentration of 0.05 wt% to 2 wt% based on the weight of the composition.

8. A method of solubilizing a halogenated diphenyl ether in a dentifrice composition comprising a source of potassium ions, wherein the source of potassium cations comprises a water soluble potassium salt selected from potassium nitrate, potassium citrate, potassium chloride, potassium bicarbonate and potassium oxalate, which potassium salt is present in the composition at a concentration of 3 wt% to 15 wt% based on the weight of the composition, and sodium lauryl sulfate present in the composition at a concentration of 0.5 wt% to 2 wt% based on the weight of the composition, the method comprising adding a cyclodextrin to the composition.

9. A method according to claim 7 wherein the halogenated diphenyl ether comprises 2,4,4'-trichloro-2'-hydroxy-diphenyl ether.

10. A method according to claim 8 wherein the cyclodextrin comprises hydroxypropyl-beta-cyclodextrin.

11. An oral composition according to claim 1 for use in a method for the treatment and prevention of bacterial plaque accumulation with reduced discomfort and pain associated with dentinal hypersensitivity, the method comprising administering the oral composition according to claim 1 to the oral cavity.

12. The oral composition of claim 1, wherein the potassium salt is at least one of potassium chloride and potassium nitrate, and/or wherein the solubilizing agent comprises 1 wt. % to 15 wt. % hydroxypropyl-beta-cyclodextrin, based on the weight of the composition

13. An oral composition according to claim 12 for use in a method for the treatment and prevention of bacterial plaque accumulation with reduced discomfort and pain associated with dentinal hypersensitivity comprising administering the oral composition according to claim 12 to the oral cavity.

## Patentansprüche

1. Eine orale Zusammensetzung, die eine oral annehmbare Trägersubstanz, eine effektive therapeutische Menge einer antibakteriellen Verbindung, eine effektive therapeutische Menge einer Quelle von Kaliumkationen, ein anionisches Tensid und ein Cyclodextrin enthält, wobei die Quelle von Kaliumkationen ein wasserlösliches Kaliumsalz umfasst, das von Kaliumnitrat, Kaliumzitrat, Kaliumchlorid, Kaliumbicarbonat und Kaliumoxalat ausgewählt ist, wobei die Konzentration des Kaliumsalzes in der Zusammensetzung 3 bis 15 Gewichtsprozent auf Basis des Gewichts der Zusammensetzung entspricht, und wobei es sich bei dem anionischen Tensid um Natriumlaurylsulfat handelt, dessen Konzentration in der Zusammensetzung 0,5 bis 2 Gewichtsprozent auf Basis des Gewichts der Zusammensetzung beträgt.

2. Die Anspruch 1 entsprechende orale Zusammensetzung, wobei die besagte antibakterielle Verbindung einen halogenierten Diphenylether umfasst, bzw. wahlweise, wobei der halogenierte Diphylether 2,4,4'-trichloro-2'-hydroxy-diphenylether umfasst, bzw. ferner wahlweise, wobei der 2,4,4'-trichloro-2'-hydroxy-diphenylether in der Zusammensetzung einer Konzentration von 0,05 bis 2 Gewichtsprozent auf Basis des Gewichts der Zusammensetzung entspricht.

3. Die Anspruch 1 entsprechende orale Zusammensetzung, wobei es sich bei der Quelle von Kaliumionen um Kaliumchlorid handelt.

4. Die Anspruch 1 entsprechende orale Zusammensetzung, wobei es sich bei dem Cyclodextrin um Hydroxypropyl-Beta-Cyclodextrin handelt.

5. Die Anspruch 1 entsprechende orale Zusammensetzung, wobei das Cyclodextrin in der Zusammensetzung einer Konzentration von 1 bis 15 Gewichtsprozent auf Basis des Gewichts der Zusammensetzung entspricht.

6. Die Anspruch 1 entsprechende orale Zusammensetzung, wobei die besagte Zusammensetzung eine Zahnpasta ist.

7. Die Anspruch 6 entsprechende orale Zusammensetzung von Zahnpasta, wobei die besagte antibakterielle Verbindung 2,4,4'-trichloro-2'-hydroxy-diphenylether umfasst, der wahlweise in der Zusammensetzung einer Konzentration von 0,05 bis 2 Gewichtsprozent auf Basis des Gewichts der Zusammensetzung entspricht.

8. Ein Verfahren zur Solubilisierung eines halogenierten Diphenylethers in einer Zahnpasta-Zusammensetzung, die eine Quelle von Kaliumionen enthält, wobei die Quelle der Kaliumkationen ein wasserlösliches Kaliumsalz umfasst, das von Kaliumnitrat, Kaliumzitrat, Kaliumchlorid, Kaliumbicarbonat und Kaliumoxalat ausgewählt ist, wobei die Konzentration des Kaliumsalzes in der Zusammensetzung 3 bis 15 Gewichtsprozent auf Basis des Gewichts der Zusammensetzung beträgt, wobei die Konzentration von Natriumlaurylsulfat in der Zusammensetzung 0,5 bis 2 Gewichtsprozent auf Basis des Gewichts der Zusammensetzung beträgt und das Verfahren umfasst, der Zusammensetzung ein Cyclodextrin hinzu zu fügen.

9. Ein Anspruch 7 entsprechendes Verfahren, wobei der halogenierte Diphenylether 2,4,4'-trichloro-2'-hydroxy-diphenylether umfasst.

10. Ein Anspruch 8 entsprechendes Verfahren, wobei das Cyclodextrin Hydroxypropyl-Beta-Cyclodextrin umfasst.

11. Eine Anspruch 1 entsprechende orale Zusammensetzung, die bei einem Verfahren zur Behandlung und Verhinderung einer Ansammlung von bakteriellem Zahnbelag benutzt wird, um die mit Dentin-Hypersensibilität verbundenen Beschwerden und Schmerzen zu reduzieren, wobei das Verfahren die Anspruch 1 entsprechende Verabreichung der oralen Zusammensetzung in der Mundhöhle umfasst.

12. Die Anspruch 1 entsprechende orale Zusammensetzung, wobei das Kaliumsalz zumindest eines von Kaliumchlorid und Kaliumnitrat ist, bzw. wobei das Solubilisierungsmittel auf Basis des Gewichts der Zusammensetzung 1 bis 15 Gewichtsprozent Hydroxypropyl-Beta-Cyclodextrin enthält.

13. Eine Anspruch 12 entsprechende orale Zusammensetzung, die bei einem Verfahren zur Behandlung und Verhinderung einer Ansammlung von bakteriellem Zahnbelag benutzt wird, um die mit Dentin-Hypersensibilität verbundenen Beschwerden und Schmerzen durch Verabreichung der Anspruch 12 entsprechenden oralen Zusammensetzung in der Mundhöhle zu reduzieren.

## Revendications

1. Une composition buccale comprenant un véhicule oralement acceptable, une quantité thérapeutique efficace d'un composé antibactérien, une quantité thérapeutique efficace d'une source de cations de potassium, un agent tensioactif anionique et une cyclodextrine, dans laquelle la source de cations de potassium comprend un sel de potassium soluble dans l'eau sélectionné à partir de nitrate de potassium, de citrate de potassium, de chlorure de potassium, de bicarbonate de potassium et d'oxalate de potassium, lequel sel de potassium est présent dans la composition à une concentration de 3 % en poids à 15 % en poids sur la base du poids de la composition, et dans laquelle l'agent tensioactif anionique est du laurylsulfate de sodium, présent dans la composition à une concentration de 0,5 % en poids à 2 % en poids sur la base du poids de la composition.

2. La composition buccale de la revendication 1, dans laquelle ledit composé antibactérien comprend un éther de diphényle halogéné, optionnellement dans laquelle l'éther de diphényle halogéné comprend de l'éther de 2,4,4'-trichloro-2'-hydroxy-diphényle, en plus optionnellement dans laquelle l'éther de 2,4,4'-trichloro-2'-hydroxy-diphényle est présent dans la composition à une concentration de 0,05 % % en poids à 2 % en poids sur la base du poids de la composition.

3. La composition buccale de la revendication 1, dans laquelle la source d'ions de potassium est du chlorure de potassium.

4. La composition buccale de la revendication 1, dans laquelle la cyclodextrine est une hydroxypropyl-béta-cyclodextrine.

5. La composition buccale de la revendication 1, dans laquelle la cyclodextrine est présente dans la composition à une concentration de 1 % en poids à 15 % en poids sur la base du poids de la composition.

6. La composition buccale de la revendication 1, dans laquelle ladite composition est un dentifrice.

7. La composition de dentifrice de la revendication 6 dans laquelle ledit composé antibactérien comprend de l'éther de 2,4,4'-trichloro-2'-hydroxy-diphényle, optionnellement présent dans la composition à une concentration de 0,05 % en poids à 2 % en poids sur la base du poids de la composition.

8. Un procédé de solubilisation d'un éther de diphényle halogéné dans une composition de dentifrice comprenant une source d'ions de potassium, dans lequel la source de cations de potassium comprend un sel de potassium soluble dans l'eau sélectionné à partir de de nitrate de potassium, de citrate de potassium, de chlorure de potassium, de bicarbonate de potassium et d'oxalate de potassium, lequel sel de potassium est présent dans la composition à une concentration de 3 % en poids à 15 % en poids sur la base du poids de la composition, et le laurylsulfate de sodium est présent dans la composition à une concentration de 0,5 % en poids à 2 % en poids sur la base du poids de la composition, le procédé comprenant l'addition d'une cyclodextrine à la composition.

9. Un procédé selon la revendication 7 dans lequel l'éther de diphényle halogéné comprend de l'éther de 2,4,4'-trichloro-2'-hydroxy-diphényle.

10. Un procédé selon la revendication 8 dans lequel la cyclodextrine comprend de la hydroxypropyl-béta cyclodextrine.

11. Une composition buccale selon la revendication 1 pour une utilisation dans un procédé pour le traitement et la prévention de l'accumulation de plaque dentaire bactérienne avec une gêne et une douleur diminués associés à l'hypersensibilité dentaire, le procédé comprenant l'administration de la composition buccale selon la revendication 1 à la cavité buccale.

12. La composition buccale de la revendication 1, dans laquelle le sel de potassium est au moins un sel de chlorure de potassium et de nitrate de potassium, et/ou dans laquelle l'agent de solubilisation comprend de 1 % en poids à 15 % en poids de hydroxypropyl-béta cyclodextrine, sur la base du poids de la composition.

13. Une composition buccale selon la revendication 12 pour une utilisation dans un procédé pour le traitement et la prévention de l'accumulation de plaque dentaire bactérienne avec une gêne et une douleur diminués associés à l'hypersensibilité dentaire, le procédé comprenant l'administration de la composition buccale selon la revendication 12 à la cavité buccale.
